Europäisches Patentamt

European Patent Office

Office européen des brevets

(19)

(11) Publication number: **0 197 485 B1**

# EUROPEAN PATENT SPECIFICATION

(12)

(45) Date of publication of patent specification: **04.03.92**   (51) Int. Cl.⁵: **A61K 7/48**

(21) Application number: **86104398.2**

(22) Date of filing: **01.04.86**

---

(54) Substantive skin care compositions comprising a polydiorganosiloxane.

---

(30) Priority: **02.04.85 US 718984**

(43) Date of publication of application:
**15.10.86 Bulletin 86/42**

(45) Publication of the grant of the patent:
**04.03.92 Bulletin 92/10**

(84) Designated Contracting States:
**BE DE FR GB IT**

(56) References cited:
**EP-A- 0 142 830
DE-A- 1 617 431
FR-A- 2 364 650
US-A- 3 392 040**

**MANUFACTURING CHEMIST AND AEROSOL
NEWS, vol. 54, no. 7, July 1983, page 59,
London, GB; "Water-repellent sunscreen"**

(73) Proprietor: **DOW CORNING CORPORATION
P.O. Box 1767
Midland Michigan 48640(US)**

(72) Inventor: **Chandra, Grish
801 Heathermoor
Midland Michigan 48640(US)**
Inventor: **Klimisch, Helen Marie
472 East Olson Road
Midland Michigan(US)**

(74) Representative: **Spott, Gottfried, Dr. et al
Patentanwälte Spott und Puschmann
Sendlinger-Tor-Platz 11
W-8000 München 2(DE)**

## Description

The present invention relates to long-lasting skin care compositions. More specifically, this invention relates to sun tan compositions which are resistant to removal from the skin by water-exposure, such as during swimming or washing.

Skin care compositions, such as face, hand and body lotions and creams, comprising one or more efficacious skin care components and, optionally, nonessential components such as diluents, surfactants, odorants, colorants, preservatives and flow-control additives are, of course, well known. So is the problem of retaining at least an effective amount of the skin care component on the skin after it has been applied thereto and exposed to water.

For example, a suntan lotion comprising an ultraviolet light-absorbing component is typically applied to the skin for protection against sunburn during extended exposure to the sun, such as during swimming, sunning and other recreational activities. However, the lotion, including the U.V. light-absorbing component, is at least partially removed from the skin during swimming and perspiration and additional lotion must be applied if maximum protection is to be maintained. It is highly desirable to be able to retain an effective amount of the U.V. light-absorbing component on the skin during repeated and/or extended exposure to water. The same can be said for other skin care compositions such as moisturizing compositions, topically applied medicament compositions and insect repellant-containing compositions.

For example, a hand lotion comprising an emollient is typically applied to hands that are frequently exposed to water in order to keep the hands from becoming red and rough. However, the emollient is soon removed by the water and must be reapplied. It is highly desirable, in this instance, to be able to retain an effective amount of the emollient on the skin during repeated and/or extended exposure to water.

Attempts have been made to increase the durability of a skin care composition on the user's skin by incorporating, in the composition, film-forming components, such as nitrocellulose (U.S. Patent No. 2,698,824), silicone resins (U.S. Patent No. 3,836,647) and alkyl esters having at least 24 carbon atoms (U.S. Patent No. 4,370,319); water-repellent liquids such as castor oil or lanolin oil (U.S. Patent No. 3,479,428) or skin-attracted compounds such as certain surfactants (U.S. Patent No. 4,335,104). Silicone fluids are frequently a required component in these durable compositions; however, the silicone fluid, when used, is not relied upon to provide skin substantivity for the composition.

Low viscosity silicone fluids have been used to prolong the effectiveness of an insect repellent, when applied to the skin; however, the skin substantivity of the composition was not considered (U.S. Patent No. 2,681,878).

The above-noted incorporation of film-forming components or water-repellent liquids into a skin care composition has been less than completely acceptable because the resulting compositions lack that certain "elegance", during and/or after application to the skin, that is appreciated by the user.

From US-PS 3 392 040 aqueous dispersions comprising an alkanoate and an organo-silicon polymer are known. The inventors of this patent found that $C_1$-$C_3$ alkyl $C_8$-$C_{14}$ alkanoates are able to dissolve polysiloxanes. At least 10 parts by weight alkanoate per 100 parts of organosiloxane are necessary to get a stable dispersion.

In FR-PS 2 364 650 it is taught an antiperspirant aerosol of the foam type, highly concentrated with respect to the active antiperspirant ingredient, which is dispensed from an aerosol container by a propellent gas, having a gas liquid volume ratio of from 10:1 to 40:1. This antiperspirant composition comprises a liquid phase of a nonvolatile miscible organic liquid of 0.1 to 30 weight percent of the final composition consisting of carboxylic acid esters of 12 to 26 carbon atoms, including linear polysiloxanes, an antiperspirant of 8 to 30 weight percent of the final composition, a liquified propellant and a solid saturated linear aliphatic carboxylic acid in an amount of 0.1 to 5.0 weight percent of the final compposition to improve the skin adherence of the applied antiperspirant.

It is an object of this invention to provide sun tan compositions which have improved resistance to removal by water. It is another object of this invention to provide sun tan compositions that provide a pleasant perception to the user during and/or after application thereof to the skin. It is a further object of this invention to provide aqueous sun tan compositions which have water-resistance after being applied to the skin.

These objects, and others which will become apparent to one of ordinary skill in the skin care composition art, are obtained by the present invention which, briefly stated, comprises incorporating into a sun tan composition comprising one or more skin care components, a skin-substantive organopolysiloxane component which has a substantially linear molecular structure. The organopolysiloxane component must be limited as to minimum viscosity in order to be an effective skin substantive component in the skin care compositions of this invention.

The present invention relates to a sun tan composition having improved resistance to removal from the skin by water-exposure, said composition comprising an amount of (A) a nonvolatile polydihydrocarbylsiloxane component having a viscosity at 25°C of at least 30 pascal-seconds and (B) a volatile polydimethylsiloxane component and one or more ultraviolet absorbing components. The composition of this invention, may be prepared by mixing a silicone-in-water emulsion comprising one or more surfactants and, as the silicone, a polydimethylsiloxane fluid component, consisting essentially of (A) from 1 to 100 percent by weight of a nonvolatile polydimethylsiloxane portion having a viscosity of at least 30 pascal-seconds at 25°C and (B) from 0 to 99 percent by weight of a volatile polydimethylsiloxane portion, and one or more ultraviolet absorbing components, the amount of said nonvolatile polydimethylsiloxane portion being sufficient to increase the skin-substantivity of at least one of said skin-care components.

Herein, the term "silicone" and the term "polydimethylsiloxane" are regarded as being synonymous and are used interchangeably. Me denotes the methyl radical. Also herein, the term "polydimethylsiloxane fluid component" denotes either a nonvolatile polydimethylsiloxane portion (A) having a viscosity of at least 30 Pa•s at 25°C or a mixture of said nonvolatile polydimethylsiloxane portion with up to 99 percent by weight of a volatile polydimethylsiloxane portion (B).

Component (A) of the compositions of this invention is a substantially linear, nonvolatile polydihydrocarbylsiloxane having a viscosity at 25°C of at least 30 Pa•s. It is necessary that the viscosity of component (A) be at least 30 Pa•s, and preferably higher, because the desired skin-substantivity enhancing effect for a skin care component is not observed for siloxanes having a lower viscosity, such as 0.5 Pa•s and 12.5 Pa•s. Preferably, Component (A) has a viscosity of at least 10 kilopascal-seconds (10 million centipoise) at 25°C.

Component (A) can be any polydihydrocarbylsiloxane having a substantially linear structure. Suitably hydrocarbyl radicals therein include alkyl radicals, having from 1 to 18 carbon atoms such as methyl, ethyl, propyl, hexyl, octyl, decyl and dodecyl; alkenyl radicals such as vinyl, allyl and hexenyl; aryl radicals, such as phenyl, benzyl and phenylethyl and cycloaliphatic radicals such as cyclohexyl. Although not being required, it is preferred that a majority, such as at least 90 percent of said hydrocarbyl radicals are methyl. The terminating radicals of the polydihydrocarbylsiloxane are not critical and can be, for example, alkyl, alkoxyl, aryl or hydroxyl. Examples of terminating radicals include methyl, phenyl, vinyl and methoxyl.

A preferred polydihydrocarbylsiloxane (A) for the purposes of this invention is a substantially linear, nonvolatile polydimethylsiloxane fluid or gum having a viscosity of at least 30 Pa•s, and preferably at least 10 kPa•s, such as 10, 20, 40 and 80 kPa•s.

The composition of this invention in addition to containing one or more skin care components and a nonvolatile polydihydrocarbylsiloxane having a viscosity of at least 30 Pa•s, also contains (B) a volatile polydimethylsiloxane. The amount of volatile polydimethylsiloxane that can be used is not critical and can range from 1 to 99 percent by weight, based on the weight of nonvolatile siloxane (A) plus volatile siloxane (B). However, to provide a pleasing sensation for the user of the compositions of this invention, it is preferred to use from 25 to 99 percent by weight, and most preferably from 50 to 90 percent by weight, of a volatile polydimethylsiloxane, particularly when the nonvolatile polydihydrocarbylsiloxane is a gum having a viscosity of at least 10 kPa•σ.

Herein the distinction between a volatile silicone and a nonvolatile polydihydrocarbylsiloxane or silicone is based on the normal boiling point. Polydimethylsiloxanes which have a normal boiling point of less than 250°C are designated as volatile silicones. All other silicones are designated as nonvolatile silicones.

Examples of volatile silicones suitable for use in this invention include cyclopolydimethylsiloxanes having the formula $(Me_2SiO)_x$ wherein x denotes 3, 4, 5 and 6 and methyl-terminated linear polydimethylsiloxanes having the formula $Me(Me_2\overline{SiO})_ySiMe_3$ wherein y has a value of 1, 2, 3 and 4.

As noted above, a nonvolatile silicone has a normal boiling point of at least 250°C. In terms of viscosity, a nonvolatile silicone for the purposes of this invention also has a viscosity at 25°C of at least 30 pascal-seconds (30,000 centipoise), such as 30, 60, 100, 1,000 Pa•s and more. For nonvolatile silicones having a viscosity exceeding 10 kPa•s it is preferred to use the well-known units of plasticity number as delineated in ASTM D926-67. Thus, for silicone viscosities ranging from 10 kPa•s to 20 kPa•s to 40 kPa•s to 80 kPa•s corresponding values of plasticity number for a substantially linear silicone will range from 130 to 146 to 165 to about 203, respectively. Correspondingly, the number average molecular weight will range from about 55,000 to about 350,000 as the viscosity ranges from 30 pascal-seconds to 100 kilopascal-seconds.

A preferred silicone component for the process of this invention and for the compositions of this invention is a bi-modal silicone component. By a bi-modal silicone component, it is meant herein a silicone that consists essentially of a substantial amount, such as for example from 25 to 99 percent by weight of a volatile silicone portion and from 1 to 75 percent by weight of a nonvolatile silicone portion having a viscosity of at least 30 pascal-seconds at 25 °C.

A highly preferred silicone component for the process and compositions of this invention is a bi-modal silicone consisting essentially of 50 to 90 parts by weight of a volatile silicone selected from octamethylcyclotetrasiloxane, decamethylcyclopentasiloxane, and their mixtures, and 10 to 50 parts by weight of a nonvolatile silicone having a viscosity of at least 10 million centipoise (10 kilopascal-seconds) at 25 °C.

The composition of this invention comprises one or more ultraviolet light absorbing component whose skin-substantivity is to be improved.

Examples of skin-protecting components include sunscreens, such as homosalate, para-aminobenzoic acid and its derivatives and oxybenzone glycerol; and insect repellents, such as N,N-dimethyltoluamide, 2-phenylcyclohexanol and 2-ethyl-1,3-hexanediol; and barriers. The composition may further comprise skin care components which are suitable for use in the compositions of this invention including skin-conditioning components, skin-protecting components, topical medicaments and cosmetic components.

Examples of skin-conditioning components include humectants, moisturizers and emollients such as mineral oils; animal oils, such as mink oil; plant oils, such as jojoba oil, aloe, cocoa butter, fatty-acids, -esters and -alcohols.

Examples of topical medicaments include stearoids; vitamins, such as tocaphenol; anesthetics such as menthol and ethyl p-aminobenzoate; antibiotics, such as fungicides; and penetrants, such as methyl salicylate.

Examples of cosmetic components include colorants, fragrances, deodorants and decolorants.

To enjoy the skin-substantivity enhancing aspects of the present invention the skin care component should be soluble in volatile silicones.

The amount of Components (A) that is used in the composition of this invention is not narrowly limited. Of course, the amount of Component (A) should be sufficient to at least increase the skin-substantivity of at least one of the ultraviolet absorbing components and, preferably, provide a feeling of "elegance" for the user of the composition. Typically, there should be at least 0.1 part by weight, and preferably 1.0 part by weight of nonvolatile polydimethylsiloxane portion per 100 parts by weight of total sun tan composition. It is also preferred that the ratio of the total amount of skin care components to the amount of nonvolatile polydimethylsiloxane portion have a value of at least 1/1 or higher, such as for example, 2/1, 4/1 or 10/1, on a weight basis.

We have noted that in the case of the sunscreen octyl p-dimethylaminobenzoate an amount of nonvolatile silicone equal to about one-half of the amount of sunscreen component provides unusual skin substantivity for the sunscreen component with respect to water-exposure.

The compositions of this invention can be of the aqueous or nonaqueous type. Nonaqueous compositions can contain, in addition to one or more skin care components and a nonvolatile polydihydrocarbylsiloxane component, one or more cosmetically suitable solvents, such as ethanol and isopropanol. However, volatile silicones are suitable solvents and present in sufficiently large amounts, may make it unnecessary to use any additional solvents.

The aqueous compositions of this invention can be prepared by any suitable manner. Typically, oil-in-water emulsion compositions are prepared by forming a liquid oil phase comprising all water-insoluble components and a liquid aqueous phase comprising all water-soluble components and thereafter combining the oil-phase and the aqueous phase, usually under the influence of heat and vigorous agitation. It is usually preferred to use only sufficient water in the aqueous phase to provide a viscous emulsion during said vigorous agitation and thereafter to dilute the viscous emulsion with additional water, as desired.

The aqueous compositions of this invention are preferably prepared by mixing a preformed silicone-in-water emulsion with an aqueous composition comprising one or more ultraviolet absorbing components, each delineated above.

Suitable silicone-in-water emulsions are provided by the methods of Narula, disclosed in commonly assigned copending applications for U.S. patent entitled, "Bi-Modal Silicone Emulsions, Silicone Emulsification Process and Emulsions Therefrom" and "Emulsification Process and Emulsions Therefrom" and filed on even date herewith. Said copending applications show how to prepare suitable emulsions of a silicone having a viscosity of at least 30 Pa•s at 25 °C.

The following examples are disclosed for the purpose of further teaching how to practice the present invention.

All parts, percentages and ratios are by weight unless otherwise indicated. Viscosities were converted from centipoise to pascal-seconds (Pa•s) for this disclosure by multiplying the former by 0.001 and are stated for 25 °C.

Two silicone-in-water emulsions were prepared and used in the following examples relating to aqueous compositions and are as follows:

Silicone Emulsion A

A bi-modal polydimethylsiloxane fluid, 100 parts, having a viscosity of about 2 kPa•s and consisting of an equal weight mixture of the volatile and nonvolatile dimethylsiloxanes disclosed in Silicone Emulsion B was thoroughly mixed with 19.1 parts of $C_8H_{17}C_6H_4(OCH_2CH_2)_{13}OH$ and 18.4 parts of $C_8H_{17}C_6H_4$-$(OCH_2CH_2)_3OH$, using a sigma blade dough mixer until homogeneous. An aqueous phase containing 15.5 parts of water, 0.3 part of PEG-120 methyl glucose dioleate and 2.5 parts of propylene glycol was slowly added to the homogeneous oil-containing phase with mixing for 100 minutes. The resulting thick, white gel was an oil-in-water emulsion having particles of 1 micrometer or less in size. This gel was diluted with 132.4 parts of distilled water, using the same mixer, to provide an oil-in-water emulsion having a siloxane content of 40%, based on siloxane plus water, and a viscosity of 2.3 Pa•s.

Silicone Emulsion B

A bi-modal polydiorganosiloxane fluid component was prepared by dissolving 13 parts of a nonvolatile polydimethylsiloxane gum having a viscosity of 40 kilopascal-seconds and a plasticity number of 165, as measured by ASTM 926-67, into 87 parts of a mixture of octamethylcyclotetrasiloxane and decamethylcyclopentasiloxane. The resulting bi-modal mixture had a viscosity of about 4 pascal-seconds.

The bi-modal mixture was mixed with 5.0 percent $C_8H_{17}C_6H_4(OCH_2CH_2)_{13}OH$ and 4.9 percent $C_8H_{17}C_6H_4(OCH_2CH_2)_3OH$ and water, 69.8 percent, was added thereto with paddle stirring. All percentages were based on the amount of bi-modal mixture. The resulting emulsion was then repeatedly passed through a colloid mill set at 1.57µm (40 mils) while maintaining the temperature of the emulsion at around 25°C. A silicone-in-water emulsion was obtained which had an average particle size of approximately 0.33µm (3300Å.)

The skin-substantivity of a material was determined by subjecting it to a Soap Scrub Regimen and/or a Water Soak Regimen, noted below. These regimens involve placing the material on an area of the volar forearm of an investigator and measuring the amount of material on the volar forearm during various portions of the regimen, using Fourier Transform-Attenuated Total Reflectance infra red spectroscopy (FT-IR). The data are expressed herein as percent of original material remaining.

The amount of test material was obtained by selecting a characteristic I.R. absorbtion for the test material which does not occur in the 1540 cm$^{-1}$ region, measuring the height of the selected band, ratioing it with the height of the skin-related Amide II band at 1540 cm$^{-1}$ and, from a calibration curve relating known amounts of test material on the skin to said ratio, determining the amount of test material on the forearm.

Soap Scrub Regimen

Test Site -          Volar Forearm
Test Area -          Rectangular Area, about 80 cm$^2$
Soap Solution -      Concentration specified, generally 0.5 wt/vol % Ivory Bar Soap Mixture
Soap Rub             - 2 passes over test area with soap solution cupped in palm of hand
Step 1     Mark test area on Forearm.
Step 2     Wash test area with soap solution using 15 rubs, rinse with 10 rubs under running tap water (cool temperature).
Step 3     Daub off excess moisture with towel, wait 1 minute.
Step 4     Hydrate skin for 1 minute using H$_2$O saturated towel held loosely over test area.
Step 5     Daub off excess moisture, wait 30 seconds.
Step 6     Run background scan of skin with ATR attachment in FT-IR. Generally use GE prism in ATR, but ZnSe prism may also be used.
Step 7     Apply test material to test area to maximum loading of 10-12 mg. on 80 cm$^2$. Generally apply test material from cyclopolydimethylsiloxane solution using paint brush or from emulsion using finger to rub in and cover test area.
Step 8     Wait 15-30 minutes for evaporation of volatile materials.
Step 9     Hydrate skin (Step 4) for 2 minutes, daub off excess moisture and wait 30 seconds.
Step 10    Run scan of test area; designate spectrum as Initial Condition.
Step 11    Wash test area with 15 soap solution rubs, rinse with 10 rubs under tap water, daub off excess moisture, wait 1 minute.
Step 12    Hydrate skin for 1 minute, daub off excess moisture, wait 30 seconds.

Step 13    Run scan of test area; designate spectrum as 1st wash condition.
Step 14    Repeat Steps 11-13 for the number of wash cycles required, generally 3 wash cycles.

Water Soak Regimen

Steps 1-10    Same as for Soap Scrub Regimen.
Step 11    Soak test area in 35°C water bath for 10 minutes, daub off excess moisture, wait 4 minutes.
Step 12    Hydrate skin for 1 minute, daub off excess moisture, wait 30 seconds.
Step 13    Run scan of test area, designate spectrum as 1st soak condition.
Step 14    Repeat Steps 11 and 13 for number of soak cycles required, generally 3 soak cycles.

Examples 1 to 4

Five suntan lotions of the oil-in-water type containing 4.44% sunscreen and various amounts of silicone gum were prepared, in each case, by preparing the following oil phase and aqueous phase and dispersing the oil phase into the aqueous phase at 75°C using a propeller stirrer, cooling the resulting emulsion to 33°C and admixing the indicated amount of Silicone Emulsion A.

Oil Phase    - Stearic acid, 5 parts; cetyl alcohol, 1.1 parts; myristyl myristate, 0.5 part; mineral oil, 5 parts; Finsolv TN®,3 parts; and octyl p-dimethylaminobenzoate, 4 parts.

Aqueous Phase    - Triethanolamine, 2 parts; Laponite XLG®, 0.5 part; Germaben II®, 1 part; and distilled water, 65.3 parts (Ex. 1); 64.41 parts (Ex. 2); 62.69 parts (Ex. 3); 57.48 parts (Ex. 4); and, 67.9 parts (Comparison).

Silicone Emulsion A    - 2.60 parts (Ex. 1); 3.49 parts (Ex. 2); 5.21 parts (Ex. 3); 10.42 parts (Ex. 4) and none (Comparison).

The five suntan lotions were subjected to the Water Soak Regimen noted above and the skin-substantivity of the octyl p-dimethylaminobenzoate was determined by measuring the intensity of its 1183 cm$^{-1}$ band in the infrared spectrum. The results summarized in Table I, show that the sunscreen agent in the compositions of this invention is 30 to 60% more substantive to the skin than in the Comparison (i) composition. In addition, the composition of this invention containing one parts of silicone gum for every two parts of octyl p-dimethylaminobenzoate (Ex. 4) displayed unusual (64 to 103%) skin-substantivity, compared to the Comparison (i) composition.

## Table I

| Composition | % Silicone Added | % Sunscreen Remaining After | | |
|---|---|---|---|---|
| | | 1st Soak | 2nd Soak | 3rd Soak |
| Ex. 1 | 0.55 | 51 | 46 | 42 |
| Ex. 2 | 0.74 | 52 | 47 | 45 |
| Ex. 3 | 1.11 | 51 | 42 | 41 |
| Ex. 4 | 2.22 | 64 | 59 | 49 |
| Comparison (i) | 0 | 39 | 29 | 25 |
| Ex. 5 | 1.4 | 55 | 39 | 35 |
| Comparison (ii) | 0 | 38 | 23 | 18 |
| Ex. 6 | 1.2 | 66 | 47 | 30 |
| Ex. 7 | 1.6 | 56 | 35 | 26 |
| Comparison (iii) | 0 | 45 | 32 | 30 |

Example 5

A composition of this invention containing 1.4% silicon gum was prepared by admixing 7.8 parts of Silicone Emulsion A with 100.2 parts of a commercial suntan lotion (Sea and Ski° Block Out® SPF 15; Sea & Ski Corporation; Philadelphia, PA). This composition, and the commercial suntan lotion with no added silicone were subjected to the Water Soak Regimen and the skin-substantivity of the sunscreen component absorbing at 1183 cm$^{-1}$ was measured. The results, shown in Table I, demonstrate that the incorporation of 1.4% of a silicone gum into this commercial suntan lotion increases the skin-substantivity of the ultraviolet light-absorbing component by 94% after 3 exposures to water-soaking.

Examples 6 and 7

A Comparison (iii) suntan lotion was prepared by preparing the following oil phase and aqueous phase and admixing the oil phase into the aqueous phase at 75°C, using a propeller stirrer and then cooling to room temperature.

Oil Phase      - Steareth-2, 1.5 parts; Brij 721®, 2.5 parts; cetyl alcohol, 1.5 parts; myristyl myristate, 0.5 part; mineral oil, 6.9 parts; Finsol® TN, 3.0 parts and octyl p-dimethylaminobenzoate, 4.0 parts.

Aqueous Phase      - Laponite XLG®, 0.5 part; Germaben®, 1 part; and water 79.5 parts.

Two compositions of this invention were prepared by mixing 18.9 parts and 28.3 parts, respectively, of Silicone Emulsion A with 100 parts of Comparison (iii) composition. The compositions of this invention had 1.2% and 1.6% silicone gum, respectively, and a sunscreen/silicone gum ratio of 2.9/1 and 2.0/1, respectively.

The comparison composition and the two compositions of this invention were subjected to the Water Soak Regimen and the skin-substantivity of the sunscreen component absorbing at 1183 cm$^{-1}$ was measured. The results, shown in Table I, further illustrate the improved skin-substantivity of a sunscreen component in the compositions of this invention.

Example 8

A Comparison (iv) moisturizing lotion was prepared by preparing the following oil phase and aqueous phase and admixing the oil phase into the aqueous phase at 75°C, using a propeller mixer and then cooling to room temperature.

Oil Phase      - Steareth-2, 1.7 parts; Brij 721®, 2.1 parts; mineral oil, 5.0 parts; acetalan, 2.0 parts; myristyl myristate, 0.25 part; cetyl alcohol, 1.25 parts and isopropyl isostearate, 2.0 parts.

Aqueous Phase      - Laponite XLG®, 0.5 part; PEG 400, 3.0 parts; Germaben II®, 1.0 part; Agidew NSO®, 4.0 parts and water, 72.2 parts.

A composition of this invention was similarly prepared except the aqueous phase contained 57.2 parts of water and 20 parts of Silicone Emulsion B was added to the emulsion at about 30°C.

These moisturizing compositions were subjected to the Soap Wash Regimen noted above and the skin-substantivity of the moisturizing component was measured by measuring the degree of skin hydration. Skin hydration was measured by noting the ratio of the Amide I/Amide II absorptions at 1649 cm$^{-}$ and 1540 cm$^{-1}$.

The Comparison (iv) lotion resulted in no change in skin hydration after 1 wash, an 8% decrease after 2 washes and a 23% decrease after 3 washed.

The composition of this invention resulted in an 8% increase in skin hydration after 1 wash, no change after 2 washes and an 18% decrease after 3 washes.

It is apparent from this example that the addition of a silicone gum to a moisturizing lotion improves the moisturizing ability of the lotion.

Examples 9 and 10

Four solutions of the sunscreen octyl p-dimethylaminobenzoate in volatile cyclopolydimethylsiloxane were prepared. A nonvolatile polydimethylsiloxane was added to three of the solutions. The four compositions, each containing about 5.8% sunscreen, were then subjected to the Water Soak Regimen and the skin-substantivity of the sunscreen component absorbing at 1183 cm$^{-1}$ was measured. The results, shown in Table II, show that silicones having a viscosity of 30 Pa•s (Example 9) and >20,000 Pa•s (Example 10) increase the skin-substantivity of the sunscreen while a silicone having a viscosity of 0.5 Pa•s (Comparison (v)) decreases said skin-substantivity, compared to Comparison (vi) composition containing no silicone.

7

### Table II

| Example | Silicone Viscosity, Pa·s | % | % Sunscreen Remaining After 1st Soak | 2nd Soak | 3rd Soak |
|---|---|---|---|---|---|
| 9 | 30 | 2.9 | 73 | 55 | 35 |
| 10 | >20,000 | 2.9 | 100 | 89 | 73 |
| Comparison (v) | 0.5 | 5.7 | 23 | 26 | 26 |
| Comparison (vi) | -- | None | 63 | 38 | 32 |

#### Examples 11 and 12

Three solutions of mink oil in volatile cyclopolydimethylsiloxane were prepared. A nonvolatile polydimethylsiloxane was added to two of the solutions. The three compositions, each containing about 5.8% mink oil, were subjected to the Soap Wash Regimen and the skin-substantivity of the mink oil was measured, using its absorption at 1746 cm$^{-1}$.

The results, shown in Table III, show that a silicone having a viscosity of at least 30 Pa·s (Example 11) increases the skin-substantivity of mink oil for at least 3 soap washes when used in an amount equal to the amount of mink oil. Example 12 shows that one part of silicone gum per 2 parts of mink oil provides some skin-substantivity for mink oil through one wash.

### Table III

| Example | Silicone Viscosity, Pa·s | % | % Mink Oil Remaining After 1st Wash | 2nd Wash | 3rd Wash |
|---|---|---|---|---|---|
| 11 | 30 | 5.8 | 43 | 30 | 23 |
| 12 | >20,000 | 2.9 | 34 | 15 | 9 |
| Comparison (vii) | -- | None | 26 | 16 | 12 |
| 13 | >20,000 | 1.0 | 68 | 54 | 44 |
| Comparison (viii) | -- | None | 54 | 39 | 33 |

#### Example 13

Two solutions of mineral oil in volatile cyclopolydimethylsiloxane were prepared and a nonvolatile silicone gum was added to one of the solutions. The two solutions were subjected to the Soap Wash Regimen and the skin-substantivity of the mineral oil was measured, using its absorption at 1461 cm$^{-1}$. The Comparison (viii) composition contained 6% mineral oil and 94% volatile cyclopolydimethylsiloxane. The composition of this invention (Example 13) contained 4% mineral oil, 1% silicone gum and 95 percent volatile cyclopolydimethylsiloxane.

The results, shown in Table III, show that mineral oil is quite resistant to removal from the skin during the Soap Wash Regimen; however, as little as one part of silicone gum per 4 parts of mineral oil substantially increases that resistance.

## Claims

1. A sun tan composition having improved resistance to removal from the skin by water-exposure said composition comprising an amount of (A) a nonvolatile polydihydrocarbylsiloxane component having a viscosity at 25°C of at least 30 pascal-seconds and (B) a volatile polydimethylsiloxane component and one or more ultraviolet light-absorbing compounds.

2. A sun tan composition according to claim 1 wherein the nonvolatile polydihydrocarbylsiloxane is a

EP 0 197 485 B1

polydimethylsiloxane.

3. A sun tan composition according to claim 1 wherein the nonvolatile polydimethylsiloxane has a viscosity of at least 10 kilopascal-seconds at 25°C and the weight ratio of nonvolatile polydimethylsiloxane to volatile polydimethylsiloxane has a value of from 1/9 to 1/1.

4. A sun tan composition according to claim 1 wherein the ultraviolet light-absorbing compound is soluble in volatile cyclopolydimethylsiloxanes.

5. A sun tan composition according to claim 3 further comprising water and one or more nonionic surfactants.

**Revendications**

1. Une composition pour bronzage au soleil ayant une résistance améliorée à l'enlèvement à partir de la peau par exposition à l'eau, ladite composition comprenant une certaine quantité de (A) un composant polydihydrocarbylsiloxane non volatil ayant une viscosité d'au moins 30 pascals-secondes à 25°C et (a) un composant polydiméthylsiloxane volatil et un ou plusieurs composés absorbant la lumière ultraviolette.

2. Une composition pour bronzage au soleil selon la revendication 1, dans laquelle le polydihydrocarbylsiloxane non volatil est un polydiméthylsiloxane.

3. Une composition pour bronzage au soleil selon la revendication 1, dans laquelle la viscosité du polydiméthylsiloxane non volatil est d'au moins 10 kilopascals-secondes à 25°C et le rapport en poids du polydiméthylsiloxane non volatil au polydiméthylsiloxane volatil est de 1/9 à 1/1.

4. Une composition pour bronzage au soleil selon la revendication 1, dans laquelle le composé absorbant la lumière ultraviolette est soluble dans les cyclopolydiméthylsiloxanes volatils.

5. Une composition pour bronzage au soleil selon la revendication 3, comprenant, de plus, de l'eau et un ou plusieurs agents tensio-actifs non ioniques.

**Patentansprüche**

1. Sonnenschutzzusammensetzung mit verbesserter Widerstandsfähigkeit gegenüber einer Entfernung von der Haut durch Einwirkung von Wasser, wobei die Zusammensetzung eine Menge von (A) einer nichtflüchtigen Polydihydrocarbylsiloxankomponente mit einer Viskosität von mindestens 30 Pascalsekunden bei 25°C und (B) einer flüchtigen Polydimethylsiloxankomponente und eine oder mehrere ultraviolettlichtabsorbierende Verbindungen umfaßt.

2. Sonnenschutzzusammensetzung nach Anspruch 1, worin das nichtflüchtige Polydihydrocarbylsiloxan Polydimethylsiloxan ist.

3. Sonnenschutzzusammensetzung nach Anspruch 1, worin das nichtflüchtige Polydimethylsiloxan eine Viskosität von mindestens 10 Kilopascalsekunden bei 25°C hat und das Gewichtsverhältnis von nichtflüchtigem Polydimethylsiloxan zu flüchtigem Polydimethylsiloxan einen Wert von 1/9 bis 1/1 hat.

4. Sonnenschutzzusammensetzung nach Anspruch 1, worin die ultraviolettlichtabsorbierende Verbindung löslich ist in flüchtigen Cyclopolydimethylsiloxanen.

5. Sonnenschutzzusammensetzung nach Anspruch 3, weiter enthaltend Wasser und ein oder mehrere nichtionische oberflächenaktive Mittel.

9